# EUROPEAN PATENT APPLICATION

(11) **EP 1 358 882 A1**
(43) Date of publication of application: **05.11.2003**
(21) Application number: 02090161.7
(22) Date of filing: 30.04.2002
(51) Int. Cl.: A61K 31/565, A61P 37/02

(54) **Use of estriol sulfamate prodrugs for the treatment of autoimmune diseases**

(71) Applicant: Schering Aktiengesellschaft, 13353 Berlin (DE)
(72) Inventor: Elger, Walter, 14195 Berlin (DE); Reddersen, Gudrun, 07749 Jena (DE); Schneider, Birgitt, 07745 Jena (DE); Von Keyserlingk, Harald, 13465 Berlin (DE); Dahlke, Frank, 14163 Berlin (DE); Schwarz, Sigfrid, 07743 Jena (DE)

(57) **Abstract**

The invention relates to the use of mono-sulfamate prodrugs of estriol, such as estriol-3-sulfamate, for the treatment of autoimmune diseases, such as multiple sclerosis (MS).

## Description

### Field of the invention

The present invention relates to the use of mono-sulfamate prodrugs of estriol, such as estriol-3-sulfamate, for the treatment of autoimmune diseases, such as multiple sclerosis (MS). The present invention further relates to methods and uses for providing a blood level of estriol as observed during pregnancy comprising orally administering a sulfamate prodrug of estriol, in particular an estriol mono-sulfamate such as estriol-3-sulfamate.

### Background of the invention

Autoimmune diseases are a type of immune pathologies that result from an uncontrolled immune response against autoantigens. The susceptibility to autoimmune diseases is affected by gender. During reproductive ages, there exists a prevalence among females to suffer from an autoimmune disease such as multiple sclerosis (MS) or rheumatoid arthritis (RA). For example, the female-to-male ratio to develop MS is 2:1 (Voskuhl et al., 2001; Neuroscientist 7: 258-270; Whitacre et al., 1999; Science 283: 1277-1278).

MS is an autoimmune disorder of the central nervous system affecting the myelin sheath of neurons and leading to demyelination and subsequent neuronal cell death. The disease is mediated by CD4+ T lymphocytes, which are specific for proteins in the myelin sheath like myelin basic protein (MBP), proteolipid protein (PLP), and myelin oligodendrocyte protein. One hypothesis is that on the basis of a genetically determined predisposition, environmental factors such as viral infections trigger the outbreak of the disease which results in an imbalance in the Th1 and Th2 population of lymphocytes, thereby promoting the accumulation of activated Th1 cells that are able to penetrate the blood-brain barrier and exert proinflammatory actions in the CNS. Demyelination of axons is in part caused by myelin-specific CD4+ lymphocytes secreting Th1 cytokines like interleukin(IL)-12, interferon gamma (IFNγ) and tumour necrosis factor alpha (TNFα). This pro-inflammatory cytokine pattern is characteristic for the cell-mediated immune response. In healthy individuals the cell-mediated Th1 immune response is in balance with the humoral Th2 immune response. The humoral immune response is mediated by the anti-inflammatory Th2 cytokines IL-4, IL-5 and IL-10.

The treatment strategies of MS are currently based on immunomodulatory treatment using interferons or glatiramer acetate. However, these treatments delay progress of disease only in some patients. Corticosteroids are used for acute treatment of relapses due to their antiinflammatory effects. Treatment with corticosteroids alleviates some acute symptoms of MS but fails to affect long-term prognosis. In addition to the known, numerous side-effects of corticosteroids, they also inhibit endogenous immunosuppressive mechanisms, rendering them unsuitable for long-term therapy. For therapy of highly active disease or of patients not responding to standard treatments including patients suffering from secondary progressive MS, immunosuppressive agents like methotrexate or cyclosporine are used. These substances are often poorly tolerated.

Therefore, there exists a need for an additional treatment strategy of MS and other Th1-mediated immune diseases.

In many autoimmune diseases, such as in MS, the Th1/Th2 balance is disturbed. Female sex hormones seem to have an influence on the regulation of this balance. During pregnancy, a shift toward a Th2 cytokine pattern has been demonstrated. An improvement of the clinical symptoms of Th1-mediated immune diseases (like MS) during pregnancy has also been observed. Especially in the third trimester of pregnancy, the rate of relapse declines in women with MS (Confavreux et al., 1998; N Engl J Med 339(5): 285-291). The decrease in disease activity appears to be due at least in part to high levels of estrogens such as estradiol and estriol, which are observed during the last trimester of pregnancy. In an animal model of MS, the Th1-mediated experimental autoimmune encephalomyelitis (EAE), it has been shown that administration of estriol at levels equal to those found in pregnancy were capable of ameliorating disease (Kim et al., 1999; Neurology 52: 1230-1238; Jansson et al., 1994; J Neuroimmunol 53: 203-207). Furthermore, it has been shown by Correale et al. (1998; J Immunol 161: 3365-3374) that the secretion of the anti-inflammatory cytokine IL-10 by CD4+ lymphocytes of MS patients is stimulated by estradiol, estrone and estriol at concentrations at a similar level as in pregnancy.

WO 01/85154 discloses a method of treating immune pathologies with low dose estrogen raising the serum concentration above basal level, but below pregnancy levels.

Because of the involvement of estrogens in the regulation of the balance between pro-inflammatory and anti-inflammatory conditions, a potential therapy for patients suffering from a Th1-mediated immune disease is to administer estrogens, in particular estriols, preferably to achieve continuous serum concentrations typically found in pregnancy.

However, the therapeutic use of estrogens is afflicted with several problems. One disadvantage of the use of estrogens in therapy is their potential to cause uterine cancer (endometrium carcinoma) or breast cancer. For example, the use of estradiol could lead to the metabolite 16alpha-hydroxyestrone, a metabolite with known tumor-promoting activity (Bradlow et al., 1985; Proc Natl Sci USA 82: 6295-6299; Kabat et al., 1997; Cancer Epidemiol Biomarkers Prev 6: 505-509).

Estriol as an active principle circumvents this problem. It is believed that estriol therapy is associated with small risks of cancer development in the human. Because of the much faster dissociation of estriol-estrogen receptor (ER) complexes than the dissociation of estradiol-ER complexes, estriol acts as a weaker and only short lasting estrogen. Therefore, estriol causes minimal endometrial proliferation. In addition, estriol displays antagonistic activity on the binding of estradiol to the receptor (Clark et al, 1984; J Steroid Biochem 20: 1005-1013) and therefore estriol seems to have a protective role opposing carcinogenic effects of estradiol. The antagonistic effects of estriol are only observed if the ratio of estriol to estradiol and estrone is 10:1, below this ratio estradiol is only partially or minimally antagonized and acts as a potent estrogen (Melamed et al., 1997; Mol Endocrinol 11: 1868-1878). This ratio is achieved in late pregnancy.

One problem encountered in the prior art is the inability to achieve continuous pregnancy blood levels of estriol with a form of administration that is comfortable for the patient. When administered orally, the bioavailability of estriol is low. To achieve comparable serum levels of estriol as after intravaginal application, ten times more estriol had to be administered orally (Head et al., 1998; Altern Med Rev 3: 101-113). Thus, estriol had to be orally administered in high doses, giving rise to possible side effects. Oral application of estriol leads to high estrogenicity in the liver. Hepatic effects include, for example, the increased synthesis of factors of the blood clotting system and angiotensinogen.

Another problem known from the prior art for oral therapy with estriol is that blood levels of estriol vary widely from patient to patient, so that general recommendations of the doses are not possible.

Problematic is also the very short half-life of estriol of about 1.5-5.3 h (Heithecker et al., 1991; Horm Res 35: 234-238). Thus, to achieve well-defined and sustained blood levels of estriol similar to those found in pregnancy, high doses of oral estriol would have to be administered at short time intervals which is not convenient for the patient and several side effects have to be taken into account.

Increase of the orally administered dose of estriol is not the way to increase the desired systemic estrogenicity. Osteoprotective properties of estriol are a quite good marker for the systemic estrogenicity of estriol. EP 0 630 248 teaches that if estriol is administered transdermally in a system which continuously releases estriol for at least 24 h and thereby a constant blood level of estriol is achieved, estriol exhibits anti-osteoporotical effects. The decisive factor for these effects is the constant estriol blood level. Although Lindsay et al. (1979; Maturitas 1: 279-285) administered orally estriol in very high doses (12 mg/day), they were not able to show the osteoprotective effects.

Low hepatic estrogenicity and a well-defined and sustained blood level of estriol can be reached by some forms of application, wherein the liver passage is avoided, for example, by injection or transdermal therapy. But these forms of administration are obviously much less desirable than oral administration with respect to patient convenience and compliance.

WO 00/01675 discloses the use of biogenic estrogen, particulary estrogen sulfamates for hormone replacement therapy.

WO 01/91797 discloses compounds, acting as a prodrug, with a sulphonamide group enabling an active agent to be taken up by the erythrocytes and/or an active agent to bind to the erythrocytes and pharmaceutical compositions containing these compounds.

WO 96/05216 discloses estra-1,3,5(10)-triene sulfamate derivatives, methods of preparing such compounds and pharmaceutical compositions containing them.

In view of the problems encountered by the prior art, a new therapeutic approach for the treatment of autoimmune diseases by achieving a well-defined and sustained blood level of estriol without the described disadvantages would be desirable.

### Object of the present invention

It is the object of the present invention to prevent or reduce the disadvantages of the prior art, i.e. to provide a new strategy for the treatment of autoimmune diseases, especially MS. In an aspect of the invention, the autoimmune disease is Th1-mediated.

It is a further object of the present invention to provide a pharmaceutical composition comprising an estriol sulfamate prodrug for providing a blood level of estriol as observed during late pregnancy such as the second or third trimester of pregnancy in a woman, preferably during the last trimester in a pregnant woman.

The pharmaceutical composition is preferably suitable for oral administration.

These objects may be achieved by an estriol sulfamate prodrug, e.g. estriol mono-sulfamate, such as estriol-3-sulfamate. The prodrugs of the invention are liver bypassing prodrugs and thereby liver-estriol interactions should be prevented. Estriol sulfamate prodrugs, for example, estriol mono-sulfamates, such as estriol-3-sulfamate, achieve well-defined and sustained blood levels of estriol. Surprisingly, a suitable blood level of estriol could be achieved with oral administration at a low dosage in larger intervals compared to the application of estriol.

Therefore, the administration of the estriol prodrugs according to the invention provides a promising novel method and use for ameliorating autoimmune diseases. The invention estriol prodrugs, such as estriol-3-sulfamate, provide blood estriol levels as observed during late pregnancy. The invention prodrugs cause a shift toward an anti-inflammatory Th2-type of immune response, and therefore, provide a promising treatment of Th1-mediated immune diseases.

### Summary of the invention

The present invention relates to the use of estriol sulfamate prodrugs for the preparation of a medicament for the treatment of an autoimmune disease, such as multiple sclerosis, in a mammal. Estriol mono-sulfamates are preferred, and the prodrug estriol-3-sulfamate is most preferred. In particular, the oral administration of low doses of estriol sulfamate prodrugs, such as estriol-3-sulfamate, results in such high and sustained estriol blood level as observed in the second or third trimester of pregnancy without any effects on hepatic functions.

In another aspect, the present invention provides a method for the treatment of an autoimmune disease such as MS in a mammal in need of such treatment, said method comprising administering a pharmaceutically effective amount of an estriol sulfamate prodrug to a mammal in need thereof. For the invention methods, estriol mono-sulfamates are preferred, and the prodrug estriol-3-sulfamate is most preferred.

### Brief description of the figures

The following figures are provided to show systemic effects of the invention estriol sulfamates as compared to estriol:
Figure 1 demonstrates the superior osteoprotective effects according to the present invention of estriol-3-monosulfonate after ovariectomy compared to estriol after oral application of various doses of the compounds. The decrease in mineral bone density after ovariectomy, as a marker for loss of bone substance is reversible if constant estriol levels are established.
Figure 2 shows that the increased excretion of deoxypyridinoline in urine as a marker for an increased bone turnover after ovariectomy was suppressed after oral administration of estriol-3-sulfamate.
Figure 3 shows the tissue selectivity of the estriol-3-monosulfonate. The uterine threshold is below the osteoprotective dose after oral application of estriol-3-sulfamate.

### Detailed description of the invention

The invention relates to methods and uses of estriol sulfamate prodrugs, such as estriol mono-sulfamates, for the treatment of autoimmune diseases, such as MS.

### Estriol mono-sulfamates are represented by the general formula ( I ) below:

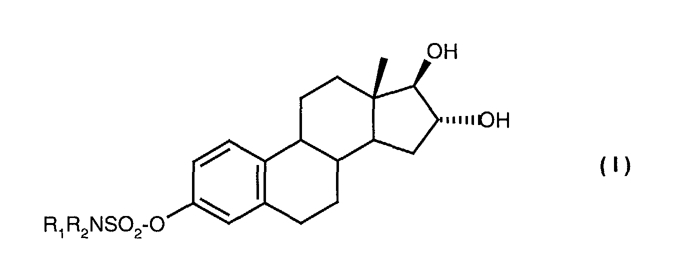

wherein
- R₁, R₂: are the same or independently are hydrogen, C₁₋₆-alkyl or CO-C₁₋₇-alkyl.

A preferred compound of this group is estriol-3-sulfamate. Although estriol-3-sulfamate is the preferred estriol mono-sulfamate for the purpose of the present invention, this does not exclude the possibility to use other suitable estriol mono-sulfamates as well.

The term "prodrug" in the context of the present invention means a biologically inactive substance, which is metabolised to the active form in the organism.

Estriol sulfamate prodrugs suitable for use in the invention and methods for their manufacture are described in WO 96/05216. In particular, the method for preparation is described in Example 15 of WO 96/05216.

The present invention provides a new therapy strategy for the treatment of autoimmune diseases, such as MS. It provides the possibility to achieve and to sustain blood levels of estriol as high as in late pregnancy. This blood level is high enough to cause an immune shift in Th1-mediated immune diseases after oral administration.

The superiority of the present invention over the prior art results from the high bioavailability of the estriol released from the sulfamate prodrug compared to the conventionally used estriol (E3). Due to the slow release of estriol from the sulfamate prodrug, high, well-defined and sustained blood levels of estriol may be reached after oral administration of relatively low dosages. Moreover, the estriol sulfamate prodrugs are thus particularly useful for the administration in large dosage intervals, another advantage of the invention. The use of relatively low dosages brings along that undesirable side effects of the continuous use of high doses of estriol can be avoided.

A favourable consequence of the administration of estriol as a sulfamate prodrug for the purpose of the present invention is that much reduced interactions with liver functions during the first-pass can be observed. This has the advantage that the compound is well tolerated. Additionally, oral administration has the advantage of improved convenience and patient compliance.

In a preferred aspect, the present invention relates to the use of estriol mono-sulfamate for the manufacture of a medicament for the treatment of an autoimmune disease. Preferably, the disease is a Th1-mediated autoimmune disease, most preferably MS. The most preferred estriol mono-sulfamate prodrug is estriol-3-sulfamate. Preferably the medicament is for the treatment in a human.

In a second preferred aspect, the present invention relates to a method for the treatment of an autoimmune disease. Preferably, the disease is a Th1-mediated autoimmune diseases, most preferably, MS. The method comprises administering estriol mono-sulfamate to a mammal, preferably a human, in need of such treatment. The estriol mono-sulfamate is most preferably estriol-3-sulfamate.

Autoimmune diseases are caused in part by T cells, which recognize a host component (autoantigen) in a specific tissue (organspecific) or in various tissues as foreign and attack that tissue. Autoimmune diseases in the context of the present invention include, but are not limited to, e.g. multiple sclerosis (MS), experimental autoimmune encephalomyelitis (EAE), rheumatoid arthritis, juvenile oligoarthritis, collagen-induced arthritis, type I diabetes mellitus, inflammatory bowel disease, Hashimoto's thyroiditis, Addison's disease, Crohn's disease, Graft-versus-host-disease, lupus disorders, and the like.

It is desirable that the prodrugs of the invention are administered in an amount sufficient to raise the serum concentration of estriol equivalent to pregnancy levels. For example, it has been observed that estriol is secreted in the order of 40 mg/24 h and circulates at a concentration of 1 - 100 ng/ml during late pregnancy in the blood (see: Katagiri et al, 1976, Am J Obstet Gynecol 272 - 280; Klopper et al, 1977, Obstet Gynecol, 459 - 461; Fischer-Rasmussen et al, 1981, Acta Obstet Gynecol Scand 417- 420).

According to the invention, estriol is administered as a sulfamate prodrug of estriol, which permits the uptake and/or binding of estriol sulfamate of the general formula (I) in erythrocytes to enhance the "first pass" effect after oral administration. While not wishing to be bound to a particular theory, it is believed that the uptake and/or binding to the erythrocytes is enabled by the -SO₂-R₁R₂ group and mediated by hemoglobin, membrane proteins and/or carboanhydratase. Thus, a depot of estriol in erythrocytes is generated. The therapeutically effective amount of estriol is received by release of the estriol by hydrolytic cleavage after liver passage. Accordingly, a high systemic estriol level may be achieved without hepatic estrogenicity side effects. Furthermore, because of the generated depot of estriol in the erythrocytes and the slow release, a well-defined and sustainable estriol blood level is obtained. The half-life of estriol in the organism is extended and the bioavailability enhanced. Accordingly, the administration in large intervals is possible.

The advantages of the invention estriol sulfamate prodrugs, such as estriol-3-O-sulfamate, are numerous, namely:
1. reduction of side effects in the liver;
2. extension of the half-life of estriol in the organism;
3. enhancement of the bioavailability; and
4. suitability for oral administration.

As a consequence, the invention prodrugs may be administered at relatively low dosages with longer intervals between the doses. Furthermore, the individual variability among patients, is decreased.

In another aspect, the present invention relates to the use of estriol sulfamate prodrugs for the manufacture of a medicament for providing a blood level of estriol as observed during late pregnancy in a woman. In a further aspect, the present invention relates to a method for providing a blood level of estriol as observed during late pregnancy in a woman comprising administering an estriol sulfamate prodrug to a mammal, preferably a human, in need thereof. Preferably, the prodrug for use in the invention or in the method of the invention is an estriol mono-sulfamate, most preferably, estriol-3-sulfamate.

The active agent suitable for the purposes of the present invention as defined above, e.g., estriol mono-sulfamate such as estriol-3-sulfamate, may be incorporated into pharmaceutical compositions according to known methods of preparing galenics.

The manufacture of the medicaments and pharmaceutical compositions for use in the invention may be performed according to methods known in the art. Commonly known and used adjuvants as well as further suitable carriers or diluents may be used. Suitable carriers and adjuvants may be such as recommended for pharmacy, cosmetics and related fields in: *Ullmann's* *Encyclopedia of Technical Chemistry,* Vol. 4, (1953), pp. 1-39; *Journal of Pharmaceutical Sciences,* Vol. 52 (1963), p. 918ff; H.v.Czetsch-Lindenwald, "Hilfsstoffe für Pharmazie und angrenzende Gebiete"; *Pharm. Ind.* 2, 1961, p.72ff; Dr. H.P. Fiedler, *Lexikon der Hilfsstoffe für Pharmazie, Kosmetik und angrenzende Gebiete*, Cantor KG, Aulendorf in Württemberg, 1971.

The administration of the estriol sulfamate prodrugs of the invention, e.g., estriol mono-sulfamate such as estriol-3-sulfamate, as a medicament may be oral, rectal or intravaginal, intrauterine, local, transdermal or parenteral. The oral application could be in the form of powder, granules, tablets, pills, pastilles, dragees, capsules, fluid extracts, tinctures and syrups. Rectal or intravaginal application could be in the form of suppositories or intrauterine devices. Local application could be in the form of suspensions or emulsions, ointments, creams or gels. The parenteral application of injectible sterile aqueous or oily solutions or suspensions could be subcutaneous, intravenous, intramuscular or percutaneous. The medicament according to the present invention may also be administered via a depot injection or an implant preparation, optionally for sustained delivery of the active agent. According to the present invention the medicament may also be administered transdermally at a controlled rate by delivering it in form of a transdermal therapeutic system like a patch.

The preferred mode of application is the oral administration. The sulfamate prodrug of estriol of the present invention is particularly suitable for oral administration.

For oral therapy, the active agent suitable for the purposes of the present invention as defined above can be admixed with commonly known and used adjuvants and carrier known in the galenic field, e.g., gum arabic, talcum, starch, sugars such as e.g. mannitose, methyl cellulose, lactose, gelatin, surface-active agents, magnesium stearate, aqueous or non-aqueous excipients, paraffin derivatives, crosslinking agents, dispersants, emulsifiers, lubricants, conserving agents and flavoring agents (e.g., ethereal oils).

The dose of the estriol sulfamate prodrug of the invention, e.g., estriol mono-sulfamate such as estriol-3-sulfamate, which has to be administered, can raise the serum concentration of estriol to at least 100 pg/ml - 50 ng/ml, preferably 1 - 10 ng/ml. A serum concentration of estriol in the range of 0,1 - 100 ng/ml is desirable, more preferably in the range of 0,1 - 10 ng/ml, most preferably in the range of 1 - 10 ng/ml. Methods of measuring the estriol serum concentration are known in the art, for example, a suitable radioimmunoassay is disclosed in Heitecker et al., 1991 (Horm Res 35: 234-238).

According to the invention, this is achieved by the oral application of the estriol sulfamate prodrugs of the invention in a cumulative dose of 5 - 500 mg per month.

Administration can take place preferably once per day, once per week or once per month. The administration can also be effected in other regular intervals, for instance every 4th, 5th, 6th, 10th etc. day.

Optionally, the pharmaceutical uses and methods according to the present invention further comprise other pharmaceutically active agents. For example, the pharmaceutically active agent may be a hormone, e.g., progesterone (gestagen), or a progesterone precursor, analog, progesterone receptor agonist or mesoprogestin. The combination of the compounds of the invention with, e.g., progesterone (gestagen) may have an additional protective effect against endometrial proliferation and other risks, associated with the long-term use of estriol. A combination with testosterone or other androgens may be needed to avoid the loss of libido due to a loss of testosterone secretation.

The treatment of autoimmune diseases, for example, Th1-mediated diseases, such as MS, with the invention estriol sulfamate prodrug may further comprise the administration of a conventional immunotherapeutic agent. The term "immunotherapeutic agent" in the context of the present invention includes, but is not limited to, immunomodulatory or immunosuppressive agents such as corticosteroids, cyclosporine, FK 506, methotrexate, azathioprine, Mitoxantrone, cyclophosphamid, glatiramer acetate copolymer-1, anti-inflammatory cytokines such as IL-4, IL-5, IL-10, IFNβ, e.g. betaferon®, cytokine-antagonists such as against IL-1, IL-2 and IL-12, TNFα, antiinflammatory PDE IV receptor antagonists e.g. mesopram, integrin α 4 antagonists and antiinflammatory chemokine antagonists such as CCR1 receptor antagonists, including antibodies, antisense oligonucleotides and soluble receptors.

The estriol sulfamate prodrugs according to the invention e.g., estriol mono-sulfamate such as estriol-3-sulfamate, and the second pharmaceutically active agent may be administered either together or separately, at the same time and/or sequentially. The mode of administration may differ between the prodrugs of the invention and the second pharmaceutically active agent.

The following examples are intended to illustrate the invention and are not to be understood as a limitation of the scope of the invention as set forth in the claims.

### Examples

The examples relate to the evaluation of the systemic effects after oral administration of various doses of estriol-3-sulfamate. Overall, the experiments show the superior systemic estrogenicity and the potency of orally administered estriol sulfamate prodrugs of the invention. Constant blood levels for estriol-3-sulfamate are demonstrated by the observed osteoprotective effects in examples 1 and 2.

### Example 1

### Materials and Methods:

Adult Wistar rats (6 months old) were ovariectomised and orally treated with estriol-3-O-sulfamate, reference compound and vehicle, respectively. Effects on bone mineral density after 28 days of treatment were determined.

### Results:

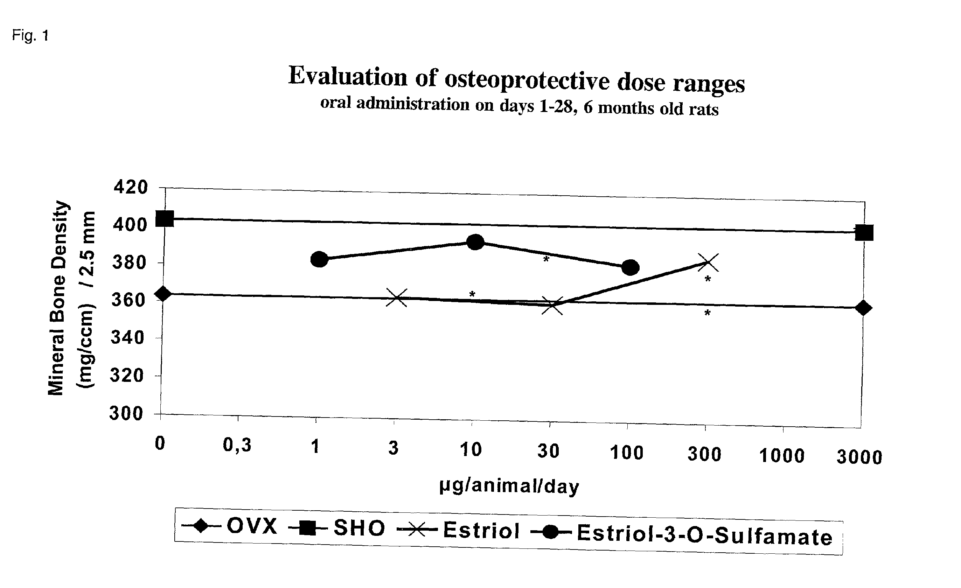

The results are shown in Figure 1. Compared to the control, ovariectomy causes a loss in bone mineral density of about 40 mg/ccm. The loss in bone mineral density could be nearly restored after oral application of 10 µg/animal/day of estriol-3-sulfamate. To achieve this effect with estriol, 30 x-fold more estriol has to be administered orally.

### Example 2

### Materials and Methods:

Adult Wistar rats (6 months old) were ovariectomised and orally treated with estriol-3-O-sulfamate, reference compound and vehicle, respectively. Effects of treatment on excretion of collagen degradation products were determined after 28 days of treatment.

### Results:

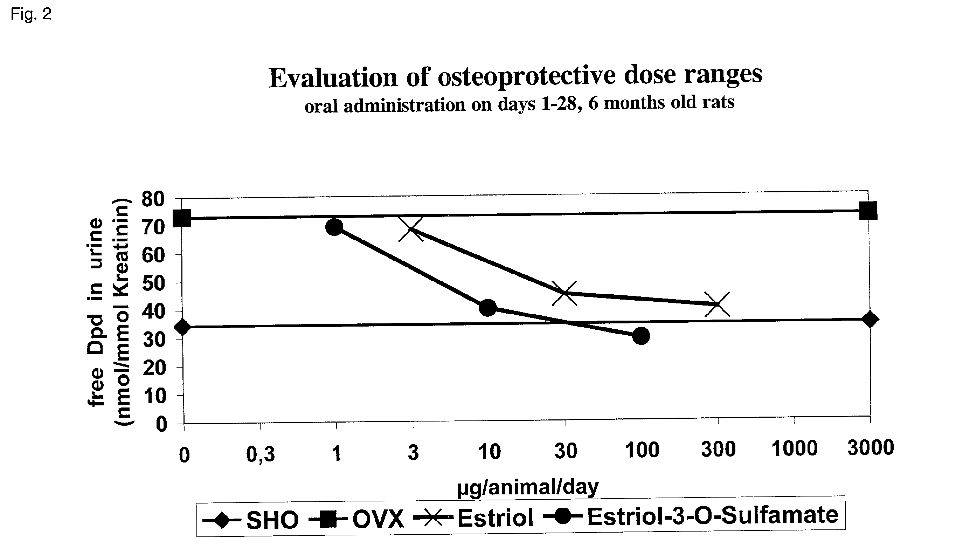

The results are shown in Figure 2. Estriol-3-sulfamate at a dose of 10 µg/animal/day causes a reduction of free deoxpyridinoline (Dpd) to a level comparable to sham operated controls. After oral administration of estriol, a reduction of free Dpd was seen at a dose of 30 µg/animal/day.

### Example 3

### Materials and Methods:

Adult Wistar rats (6 months old) were ovariectomised and orally treated with estriol-3-O-sulfamate, reference compound and vehicle, respectively. Effects of treatment on uterine weight after 28 days of treatment were determined.

### Results:

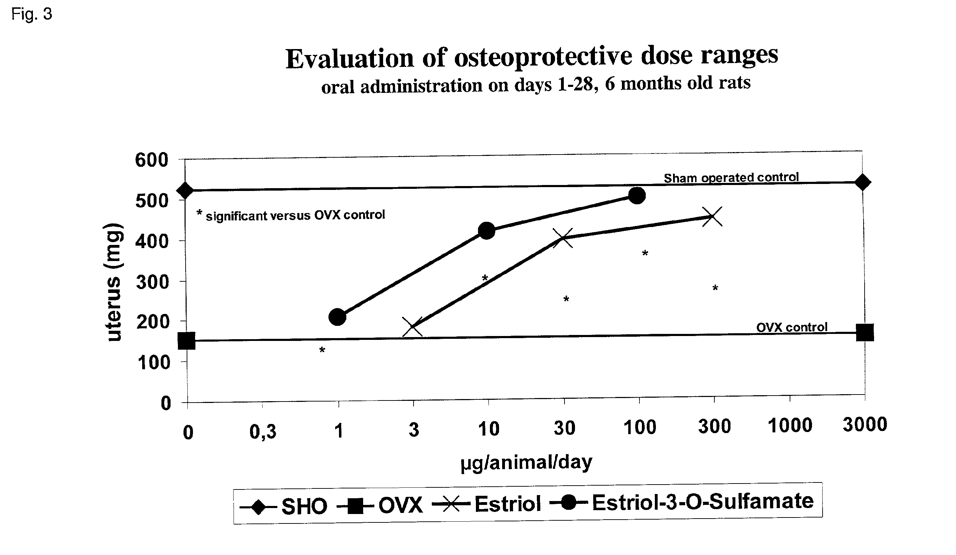

The results are shown in Figure 3. Compared to the control, ovariectomy causes a reduction of uterine weight of about 333 mg. To increase the uterine weight to basal level 100 µg/animal/day estriol-3-sulfamate have to be administered, ten times more than to show osteoprotective effects. Estriol-3-sulfamate shows also tissue selectivity by bone protection effects below the uterine threshold. To achieve the same result after oral application of estriol, 300 µg/animal/day has to be administered.

### Conclusion based on Examples 1, 2 and 3:

According to the invention, a well-established and sustained estriol blood level is fulfilled after oral application of estriol-3-sulfamate. Estriol has no bone sparing effect at doses used for oral application of estriol-3-sulfamate. The estriol sulfamate had a much higher systemic estrogen activity than the estriol after oral application. Based on the results of the uterine weight test, estriol-3-sulfamate is also more potent (about 3-fold) than estriol administered orally.

## Claims

1. Use of an estriol mono-sulfamate according to formula I 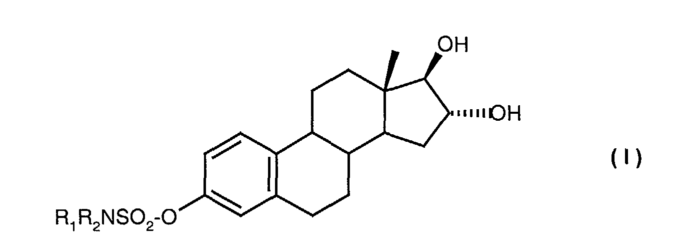 wherein
R₁, R₂ are the same or independently are hydrogen, C₁₋₆-alkyl or CO-C₁₋₇-alkyl,
for the manufacture of a medicament for the treatment of an autoimmune disease in a mammal.

2. Use according to claim 1, wherein the estriol mono-sulfamate is estriol-3-O-sulfamate.

3. Use according to claims 1 or 2, wherein the mammal is a human.

4. Use according to any one of claims 1 to 3, wherein the autoimmune disease involves a Th1-mediated immune response.

5. Use according to any one of claims 1 to 4, wherein the disease is selected from the group consisting of multiple sclerosis, rheumatoid arthritis, juvenile oligoarthritis, type I diabetes mellitus, inflammatory bowel disease, Hashimoto's thyroiditis, Addison's disease, lupus disorders, acute graft-versus-host disease, Crohn's disease.

6. Use according to any one of claims 1 to 5, wherein the disease is multiple sclerosis.

7. Use according to any one of claims 1 to 6, wherein the estriol mono-sulfamate is administered in a cumulative dose of 5 - 500 mg per month.

8. Use according to any one of claims 1 to 7, wherein treatment further comprises the administration of a second therapeutic agent.

9. Use according to any one of claims 1 to 8, wherein the medicament is to be administered orally.

10. Use of estriol mono-sulfamate for the manufacture of a medicament for providing a blood level of estriol as observed during the late pregnancy in a woman.

11. Use according to claim 10, wherein the estriol mono-sulfamate is estriol-3-O-sulfamate.

12. Use according to claims 10 or 11, wherein the estriol blood level is between 100 pg/ml and 50 ng/ml.

13. Use according to any one of claims 10 to 12, wherein the medicament is to be administered orally.
